Europäisches Patentamt

European Patent Office

Office européen des brevets

(19)

(11) Veröffentlichungsnummer: **0 229 204**
**B1**

(12) # EUROPÄISCHE PATENTSCHRIFT

(45) Veröffentlichungstag der Patentschrift:
**04.01.89**

(51) Int. Cl.⁴: **A61M 5/32**

(21) Anmeldenummer: **86100315.0**

(22) Anmeldetag: **11.01.86**

(54) Spritze für medizinische Zwecke.

(43) Veröffentlichungstag der Anmeldung:
**22.07.87 Patentblatt 87/30**

(45) Bekanntmachung des Hinweises auf die Patenterteilung:
**04.01.89 Patentblatt 89/1**

(84) Benannte Vertragsstaaten:
**AT BE CH DE FR GB IT LI LU NL SE**

(56) Entgegenhaltungen:
**EP-A- 0 022 977**
**EP-A- 0 096 314**
**FR-A- 2 510 892**
**US-A- 3 370 588**
**US-A- 3 381 813**
**US-A- 3 865 236**

(73) Patentinhaber: **Vetter, Helmut, Marienplatz 81,
D-7980 Ravensburg(DE)**

(72) Erfinder: **Vetter, Helmut, Marienplatz 81,
D-7980 Ravensburg(DE)**
Erfinder: **Geprägs, Peter, Judithaweg 6,
D-7987 Weingarten(DE)**

(74) Vertreter: **Fay, Hermann, Dipl.-Phys. Dr. et al,
Ensingerstrasse 21 Postfach 1767, D-7900 Ulm
(Donau)(DE)**

## Beschreibung

Die Erfindung betrifft eine Spritze für medizinische Zwecke mit einem Spritzenzylinder und darin verschiebbarem Spritzenkolben sowie mit einer Kanülenschutzkappe, die auf ein am Spritzenkolben endseitig angeordnetes, mit einem Außenbund versehenes Nadelansatzstück dichtend aufsteckbar ist.

Spritzen dieser Art, die bereits vollständig fertig konfektioniert, insbes. also bereits mit der Kanüle versehen und gegebenenfalls auch bereits befüllt in den Handel kommen, werden auch als sog. Fertigspritze bezeichnet. Bei solchen Fertigspritzen muß sowohl der Inhalt des Spritzenzylinders wie auch die Kanüle bis zur Andwendung steril gehalten werden. Dazu ist in bisher üblicher Ausführungsform das Nadelansatzstück mit einem Konus versehen, auf welchen die Kanülenschutzkappe aufgeschoben ist, wobei im übrigen die Kanülenspitze in die Kanülenschutzkappe eingestochen ist, wodurch eine zusätzliche Abdichtung der Fertigspritze im Bereich der Kanülenspitze erfolgt.

Bei der automatischen Herstellung und Verarbeitung solcher Fertigspritzen, insbes. beim Waschvorgang und der Sterilisation muß unmittelbar im Anschluß an diese Vorgänge die Kanülenschutzkappe dichtend auf das Nadelansatzstück aufgesteckt werden. Die Kanülenschutzkappe wird daher zunächst nur lose auf den Konus aufgeschoben, damit während der Reinigungsvorgänge, insbes. der Sterilisation mit Dampf, Gas oder dergl. die Kanüle und über die Kanülenspitze auch der Innenraum der Kanüle ebenso der Sterilisation mit unterworfen werden. Sitzt dabei die Kanülenschutzkappe bereits zu fest auf dem Konus, so kann wegen der dann schon auftretenden Abdichtung keine ausreichende Reinigung dieses Bereichs mehr erfolgen. Ist die Kanülenschutzkappe dagegen zu lose auf den Konus aufgeschoben, so kann sie sich während des Transports und insbesondere bei der Evakuierung ablösen.

Aus der US-A 3 865 236 ist bereits eine Spritze bekannt, deren Kanülenschutzkappe mit einem selbsttätig arbeitendem Ventil versehen ist. Dieses Ventil ist von einem in der Wand der Kanülenschutzkappe angeordneten und sich durch diese hindurch erstreckenden Schlitz gebildet, der normalerweise geschlossen ist. Während des Sterilisierungsvorgangs, insbes. bei abwechselnder Behandlung mit Dampf und Vakuum, öffnet das Ventil unter der auftretenden Druckdifferenz und verhindert so einen Überdruck im Innern der Kanülenschutzkappe, durch welchen diese vom Nadelansatzstück abgehoben werden könnte. Nachteilig hierbei ist jedoch, daß eine Reinigung bzw. Sterilisation des Innenraums der Kanülenschutzkappe nur über die schmale Ventilöffnung und durch Aufrechterhaltung einer Druckdifferenz erfolgen kann, was in der Praxis nicht möglich ist.

Der Erfindung liegt die Aufgabe zugrunde, eine Spritze der eingangs genannten Art so auszubilden, daß sie im Rahmen einer automatischen Verarbeitung vor dem Reinigungs- und Sterilisiervorgang bereits mit der Kanülenschutzkappe versehen werden kann, wobei sichergestellt wird, daß auch die Kanüle selbst der Sterilisation unterworfen wird, ohne daß andererseits die Gefahr besteht, daß die Kanülenschutzkappe sich während des Verarbeitungsprozesses von der Spritze lösen kann.

Diese Aufgabe wird nach der Erfindung dadurch gelöst, daß die Kanülenschutzkappe zwei in axialer Richtung hintereinander angeordnete, jeweils einen Rastsitz für den Außenbund bildende Ringnuten von im wesentlichen gleichem Durchmesser aufweist, wobei in den zwischen den beiden Ringnuten gebildeten Raum einer oder mehrere sich zur Außenseite der Kanülenschutzkappe hin erstreckende Kanäle münden.

Der durch die Erfindung erreichte Fortschritt besteht im wesentlichen darin, daß die Kanülenschutzkappe zunächst nur soweit auf das Nadelansatzstück aufgeschoben wird, bis der Außenbund in die in Aufschubrichtung vorgelagerte erste Ringnut einrastet, wodurch das Innere der Kanülenschutzkappe und damit auch die Kanüle selbst über die sich zur Außenseite der Kanülenschutzkappe hin erstreckenden Kanäle mit einer vorgegebenen, definierten Querschnittsfläche mit der Außenseite in Verbindung stehen. Damit ist eine zuverlässige Reinigung und Sterilisation auch dieses Bereiches gewährleistet. Andererseits kann wegen des Rastsitzes die Kanülenschutzkappe weder beim Transport der Spritzen noch beim Evakuieren abfallen, zumal in letzterem Fall der definierte und fest vorgegebene Öffnungsquerschnitt der Kanäle für ein hinreichend schnelles Abströmen des Gasvolumens aus der Kanülenschutzkappe Sorge trägt. Nach erfolgter Sterilisation muß die Kanülenschutzkappe lediglich soweit vorgeschoben werden, bis der Außenbund in die zweite Ringnut greift, wodurch das Innere der Kanülenschutzkappe gegen das Nadelansatzstück vollständig definitiv abgedichtet ist. Im Ergebnis kann somit der durch abgehende oder schlecht sitzende Kanülenschutzkappen auftretende Ausschuß während der Verarbeitungsphase erheblich verringert werden. Ferner ist damit erstmals eine Validierung der Sterilisation des Innenraums der Kanülenschutzkappe sowie der Kanüle selbst möglich, während bisher ein solcher Nachweis aufgrund eines fehlenden oder nicht kontrollierbaren Gasaustausches zwischen der Kanülenschutzkappe und der Umgebung nicht möglich war.

In einer bevorzugten Ausführungsform der Erfindung sind die Kanäle von Axialnuten gebildet, die in der Mantelfläche der in Aufsteckrichtung der Kanülenschutzkappe ersten Ringnut in axialer Richtung zum offenen Ende der Kanülenschutzkappe hin verlaufen. Zweckmäßigerweise sind hierbei insgesamt vier gleichmäßig über den Umfang verteilt angeordnete Kanäle vorgesehen, wodurch einerseits ein ausreichender Kanalquerschnitt und andererseits ein hinreichender fester Sitz des Ringbundes in der Ringnut gewährleistet ist.

Um das Aufstecken der Kanülenschutzkappe auf das Nadelansatzstück zu erleichtern, empfiehlt es sich, daß das kanülenseitige Ende des Nadelansatzstücks die Gestalt einer sich axial zur Kanüle hin verjüngenden Vollkegelfläche besitzt, deren Mantelfläche unmittelbar in den Außenbund übergeht. In der Regel wird die Kanülenschutzkappe mehr oder weniger vollständig aus elastischem Material bestehen. In jedem Fall empfiehlt es sich jedoch, daß die in Aufsteckrichtung der Kanülenschutzkappe den Ringnuten jeweils vorgelagerten Ringlippen von gummielastischem Material gebildet sind, um während des Aufsteckens der Kanülenschutzkappe eine hinreichende Verformbarkeit der Ringlippen zu ermöglichen.

Weiter empfiehlt es sich im Rahmen der Erfindung, daß die Kanülenschutzkappe in dem zur Aufnahme der Kanüle vorgesehenen, an die zweite Ringnut angrenzenden Bereich sich innenseitig hohlkegelförmig verjüngt und die Länge des hohlkegelförmigen Bereichs so bemessen ist, daß die Kanülenspitze die Spitze des Hohlkegels eben berührt, wenn der Außenbund in die erste Ringnut eingerastet ist. Somit ist die Kanülenspitze während des Wasch- und Sterilisationsvorgangs frei zugänglich, so daß auch das Kanüleninnere in den Reinigungsvorgang mit einbezogen ist. Ferner sollte der gegenseitige Abstand der beiden Ringnuten zweckmäßigerweise wenigstens so groß sein, wie die Länge des keilförmig angeschliffenen Endes der Kanülenspitze, so daß nach dem endgültigen Aufstecken der Kanülenschutzkappe und Einrasten des Ringbundes in die zweite Ringnut die Kanülenspitze vollständig in die Kanülenschutzkappe eingestochen ist.

Im folgenden wird die Erfindung an einem in der Zeichnung dargestellten Ausführungsbeispiel näher erläutert; es zeigen:

Fig. 1 den Gegenstand nach der Erfindung in für die Sterilisation vorgesehenem Zustand,
Fig. 2 den Gegenstand nach Fig. 1, jedoch nach Abschluß der Sterilisation bzw. vor Beginn der Weiterverarbeitung der sterilisierten Objekte,
Fig. 3 einen Schnitt durch den Gegenstand nach Fig. 1 längs der Linie A-A.

Die in der Zeichnung dargestellte Spritze ist für medizinische Zwecke vorgesehen und besteht aus einem in der Zeichnung nur schematisch angedeuteten Spritzenzylinder 1 mit einem darin verschiebbaren Spritzenkolben 2. Die Spritze ist ferner mit einer Kanülenschutzkappe 3 versehen, die auf ein am Spritzenzylinder 1 endseitig angeordnetes Nadelansatzstück 4 dichtend aufsteckbar ist.

Im einzelnen ist das Nadelansatzstück 4 mit einen Außenbund 5 versehen. Die Kanülenschutzkappe 3 weist zwei in ihrer Längsachse 6 hintereinander angeordnete, jeweils einen Rastsitz für den Außenbund 5 bildende Ringnuten 7, 8 auf, die im wesentlichen den gleichen Durchmesser besitzen. In den zwischen den beiden Ringnuten 7, 8 gebildeten Raum 9 münden mehrere Kanäle 10, die sich zur Außenseite der Kanülenschutzkappe 3 hin erstrecken.

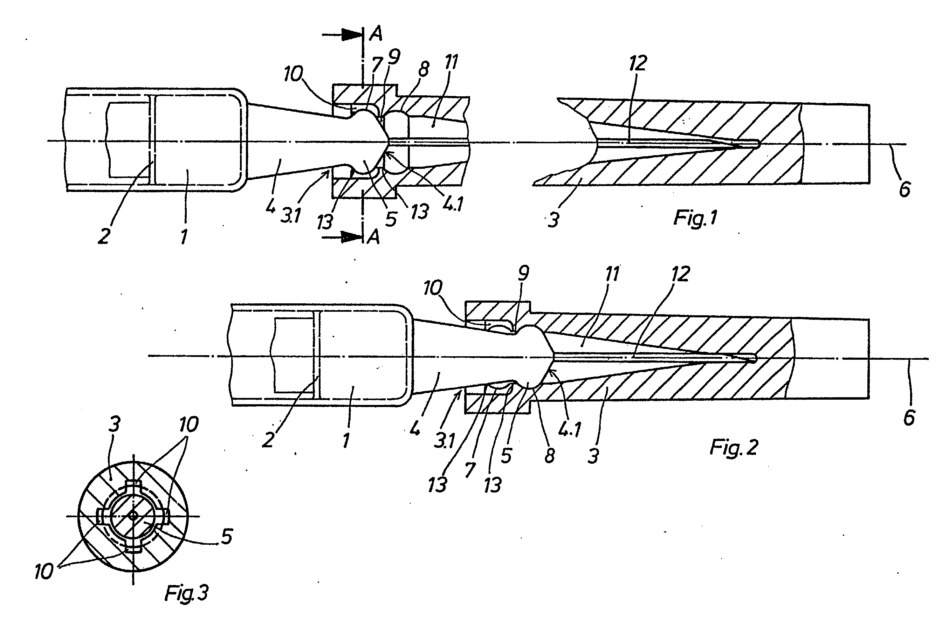

Solange die Kanülenschutzkappe 3 gemäß Fig. 1 nicht vollständig auf das Nadelansatzstück 4 aufgeschoben ist, so daß der Außenbund 5 in die in Aufsteckrichtung der Kanülenschutzkappe 3 erste Ringnut 7 einrastet, steht der Innenraum 11 der Kanülenschutzkappe 3 über die Kanäle 10 mit dem Äußeren der Kanülenschutzkappe 3 in Verbindung.

Während des automatisch ablaufenden Sterilisationsvorganges ist die Kanülenschutzkappe 3 somit einerseits bereits fest mit der Spritze verbunden, andererseits besteht eine hinsichtlich ihrer Querschnittsfläche genau bestimmte Verbindung zum Innenraum der Kanülenschutzkappe 3 und der Kanüle 12 selbst, so daß auch dort die notwendige und gewünschte Sterilisation erfolgen kann. Nach Abschluß dieser Vorgänge wird die Kanülenschutzkappe 3 weiter auf das Nadelansatzstück 4 aufgeschoben, bis der Außenbund 5 in die zweite Ringnut 8 gemäß Fig. 2 einrastet und dadurch die Verbindung des Innenraums 11 der Kanülenschutzkappe 3 nach außen hin unterbrochen wird. Hierdurch ist erstmals eine Validierung der Sterilisation des Innenraums 11 der Kanülenschutzkappe 3 sowie der Kanüle 12 möglich, und Kanüle 12 sowie Innenraum der Kanülenschutzkappe 3 können steril dicht gehalten werden.

Die Kanäle 10 sind im einzelnen, wie sich insbes. aus Fig. 3 ergibt, von Axialnuten gebildet, die in der Mantelfläche der in Aufsteckrichtung der Kanülenschutzkappe 3 ersten Ringnut 7 in axialer Richtung zum offenen Ende 3.1 der Kanülenschutzkappe 3 hin verlaufen. Die Anzahl und Größe der Axialnuten kann weitgehend beliebig gewählt sein und insoweit den Erfordernissen angepaßt werden, solange ein ausreichender Rastsitz des Außenbundes 5 in der ersten Ringnut 7 gewährleistet ist. Wie die Fig. 3 erkennen läßt, sind im Ausführungsbeispiel insgesamt vier gleichmäßig über der Umfang verteilt angeordnete Kanäle vorgesehen.

Um das Aufsetzen der Kanülenschutzkappe 3 auf das Nadelansatzstück 4 zu erleichtern, besitzt das kanülenseitige Ende des Nadelansatzstücks 4 die Gestalt einer sich axial zur Kanüle 12 hin verjüngenden Vollkegelfläche 4.1. Dabei geht deren Mantelfläche unmittelbar in den Außenbund 5 über. Ferner sind auch die in Aufsteckrichtung der Kanülenschutzkappe 3 den Ringnuten 7, 8 jeweils vorgelagerten Ringlippen 13 zweckmäßigerweise von gummielastischem Material gebildet, um das automatische Zentrieren und Aufstecken der Kanülenschutzkappe 3 zu vereinfachen.

Die Kanülenschutzkappe 3 ist im einzelnen so ausgebildet, daß sie sich in dem zur Aufnahme der Kanüle 12 vorgesehenen, an die zweite Ringnut angrenzenden Innenraum 11 hohlkegelförmig verjüngt. Dabei ist die Länge des hohlkegelförmigen Innenraums 11 so bemessen, daß die Kanülenspitze die Spitze des Hohlkegels eben berührt, wenn der Außenbund 5 entsprechend der Fig. 1 in die erste Ringnut 7 eingerastet ist. Dadurch ist sichergestellt, daß im Verlauf des Reinigungs- und Sterilisationsprozesses auch das Kanüleninnere mit einbezogen wird. Der gegenseitige Abstand der beiden Ringnuten 7, 8 sollte wenigstens so groß sein wie die Länge des keilförmig angeschliffenen Endes der Kanülenspitze, damit nach dem vollständigen Aufschieben der Kanülenschutzkappe 3 die Spitze der Kanüle 12 vollständig in diese eindringt und somit eine vollständinge Abdichtung auch an der Kanülenspitze erfolgt.

## Patentansprüche

1. Spritze für medizinische Zwecke mit einem Spritzenzylinder (1) und darin verschiebbarem Spritzenkolben (2) sowie mit einer Kanülenschutzkappe (3), die auf ein am Spritzenzylinder (1) endseitig angeordnetes, mit einem Außenbund (5) versehenes Nadelansatzstück (4) dichtend aufsteckbar ist, dadurch gekennzeichnet, daß die Kanülenschutzkappe (3) zwei in axialer Richtung hintereinander angeordnete, jeweils einen Rastsitz für den Außenbund (5) bildende Ringnuten (7, 8) von im wesentlichen gleichem Durchmesser aufweist, wobei in den zwischen den beiden Ringnuten (7, 8) gebildeten Raum (9) einer oder mehrere sich zur Außenseite der Kanülenschutzkappe (3) hin erstreckende Kanäle (10) münden.

2. Spritze nach Anspruch 1, dadurch gekennzeichnet, daß die Kanäle (10) von Axialnuten gebildet sind, die in der Mantelfläche der in Aufsteckrichtung der Kanülenschutzkappe (3) ersten Ringnut (7) in axialer Richtung zum offenen Ende (3.1) der Kanülenschutzkappe (3) hin verlaufen.

3. Spritze nach Anspruch 2, dadurch gekennzeichnet, daß insgesamt vier gleichmäßig über den Umfang verteilt angeordnete Kanäle (40) vorgesehen sind.

4. Spritze nach den Ansprüchen 1 bis 3, dadurch gekennzeichnet, daß das kanülenseitige Ende (4.1) des Nadelansatzstückes (4) die Gestalt einer sich axial zur Kanüle (12) hin verjüngenden Vollkegelfläche besitzt, deren Mantelfläche unmittelbar in den Außenbund (5) übergeht.

5. Spritze nach den Ansprüchen 1 bis 4, dadurch gekennzeichnet, daß die in Aufsteckrichtung der Kanülenschutzkappe (3) den Ringnuten (7, 8) jeweils vorgelagerten Ringlippen (13) von gummielastischem Material gebildet sind.

6. Spritze nach den Ansprüchen 1 bis 5, dadurch gekennzeichnet, daß die Kanülenschutzkappe (3) in dem zur Aufnahme der Kanüle (12) vorgesehenen, an die zweite Ringnut 8 angrenzenden Innenraum (11) sich innenseitig hohlkegelförmig verjüngt und die Länge des hohlkegelförmigen Innenraums (11) so bemessen ist, daß die Kanülenspitze die Spitze des Hohlkegels gerade eben berührt, wenn der Außenbund (5) in die erste Ringnut (7) eingerastet ist.

7. Spritze nach Anspruch 6, dadurch gekennzeichnet, daß der gegenseitige Abstand der beiden Ringnuten (7, 8) wenigstens so groß ist wie die Länge des keilförmig angeschliffenen Endes der Kanülenspitze.

## Claims

1. A hypodermic syringe for medical purposes, having a syringe cylinder (1) and a syringe plunger (2) movable therein, and having a hollow needle protection cap (3), which may be mounted so as to form a seal on a needle joining piece (4) provided with an outer collar (5) and disposed at the end of the syringe cylinder (1), characterised in that the hollow needle protection cap (3) has two annular grooves (7, 8) of substantially equal diameter disposed one behind the other in the axial direction and each forming a snap fit for the outer collar (5), one or more channels (10) extending towards the outside of the hollow needle protection cap (3) and opening out in the space (9) formed between the two annular grooves (7, 8).

2. A hypodermic syringe according to claim 1, characterised in that the channels (10) are formed by axial grooves extending towards the open end (3.1) of the hollow needle protection cap (3) in the axial direction in the lateral surface of the first annular groove (7) in the direction of mounting of the hollow needle protection cap (3).

3. A hypodermic syringe according to claim 2, characterised in that, in all, four channels (40) are provided, evenly distributed over the circumference.

4. A hypodermic syringe according to claims 1 to 3, characterised in that the hollow needle-side end (4.1) of the needle joining piece (4) has the shape of a cone tapering axially towards the hollow needle (12) and whose generated surface passes directly into the outer collar (5).

5. A hypodermic syringe according to claims 1 to 4, characterised in that the annular lips (13) disposed in each case in front of the annular grooves (7, 8), seen in the direction of mounting of the hollow needle protection cap (3), are formed of rubber elastic material.

6. A hypodermic syringe according to claims 1 to 5, characterised in that the hollow needle protection cap (3) tapers in a hollow conical shape on the inside in the internal space (11) provided for receiving the hollow needle (12) and bordering the second annular groove (8), and the length of the hollow conical internal space (11) is dimensioned such that the point of the hollow needle just touches the point of the hollow cone when the outer collar (5) is locked into the first annular groove (7).

7. A hypodermic syringe according to claim 6, characterised in that the mutual distance between the two annular grooves (7, 8) is at least as great as the length of the end of the hollow needle point, which is ground into a wedge shape.

## Revendications

1. Seringue médicale comportant un cylindre (1) et un piston coulissant (2), ainsi qu'un capuchon (3) de protection de canule qui peut être enfoncé de manière étanche sur un embout (4) pour aiguille muni d'un collet extérieur (5) et situé sur le cylindre (1) de la seringue du côté extrémité, caractérisée en ce que le capuchon de protection (3) de canule comporte deux gorges annulaires (7, 8) de diamètre à peu près égal, disposées l'une derrière l'autre en direction axiale, formant respectivement un siège d'encliquetage pour le collet extérieur (5), un ou plusieurs canaux (10) s'étendant du côté extérieur du capuchon de protection (3) de la canule débouchant dans l'espace (9) ménagé entre les deux gorges annulaires (7, 8).

2. Seringue selon la revendication 1, caractérisée en ce que les canaux (10) sont formés par des rainures axiales qui s'étendent dans la surface de l'enveloppe de la première gorge annulaire (7) dans la direction d'enfoncement du capuchon de protection (3) en direction axiale vers l'extrémité ouverte (3.1) du capuchon de protection (3) de la canule.

3. Seringue selon la revendication 2, caractérisée en ce qu'il est prévu en tout quatre canaux (10) uniformément répartis sur la périphérie.

4. Seringue selon l'une quelconque des revendications 1 à 3, caractérisée en ce que l'extrémité (4.1) côté canule de l'embout (4) de l'aiguille a la forme d'une surface totalement conique se rétrécissant axialement en direction de la canule (12) et dont la surface se raccorde directement au collet extérieur (5).

5. Seringue selon l'une quelconque des revendications 1 à 4, caractérisée en ce que des lèvres annulaires (13) ménagées respectivement dans la direction d'enfoncement du capuchon de protection (3) de la canule en avant des gorges annulaires (7, 8) sont constituées par un matériau élastique en caoutchouc.

6. Seringue selon l'une quelconque des revendications 1 à 5, caractérisée en ce que le capuchon de protection (3) de la canule se rétrécit intérieurement de forme conique creuse dans l'espace intérieur (11) raccordé à la seconde gorge annulaire (8) prévue pour recevoir la canule (12) et en ce que la longueur de l'espace intérieur (11) de forme conique creuse est dimensionnée de telle sorte que la pointe de la canule touche exactement le sommet du cône creux lorsque le collet extérieur (5) est encliqueté dans la première gorge annulaire (7).

7. Seringue selon la revendication 6, caractérisée en ce que la distance mutuelle des deux gorges annulaires (7, 8) est au moins aussi grande que la longueur de l'extrémité de la pointe de la canule taillée en forme de coin.

Fig. 1

Fig. 2

Fig. 3